# EUROPEAN PATENT APPLICATION

(11) **EP 4 115 878 A1**
(43) Date of publication of application: **11.01.2023**
(21) Application number: 21305967.8
(22) Date of filing: 09.07.2021
(51) Int. Cl.: A61K 9/20, A61K 31/00, B29C 64/00, B33Y 70/00

(54) **STARCH BASED PRINTABLE MATERIALS**

(71) Applicant: ROQUETTE FRERES, 62136 Lestrem (FR)
(72) Inventor: LUI, Yuan-Siang, 138667 SINGAPORE (SG); CHOW, Keat-Theng, 138667 SINGAPORE (SG); GOKHALE, Rajeev, 138667 SINGAPORE (SG); HAEUSLER, Olaf, 59270 FLETRE (FR)
(74) Representative: Plasseraud IP

(57) **Abstract**

The instant invention relates to a printable material for Hot Melt Extrusion-based 3D printing (HME-3DP) comprising a pregelatinized cross-linked starch and hydroxypropyl methyl cellulose (HPMC). It also relates to a process for 3D printing using the same. It also relates to products, in particular controlled release solid dosage forms, obtained thereof by 3D printing

## Description

### Technical Field

The present invention relates to printable materials, in particular for use in the 3D printing of solid dosage forms.

### Background Art

3D printing (3DP), also known as additive manufacture (AM), is a process for constructing 3D physical objects from digital models through the successive layer-by-layer deposition of materials such as plastic, metal, ceramics, or even living cells.

Apart from many applications unrelated to healthcare, 3DP is currently used or under research for oral drugs, implantable drug delivery devices, tissue bioprinting, devices such as prosthesis and even food products.

When compared to traditional drug manufacturing, it was observed that 3DP is capable of producing reasonably priced, on-demand, patient tailored drugs and/or an increased product complexity. The shapes and internal structures of current drug products are limited by current technologies. When using additive manufacturing, these characteristics can be as complex as one can imagine, and this could prove useful especially for multiple drug release profiles embedded in the same dosage form. Some of the goals are minimising side effects, by achieving near-zero-order release by printing toroidal, cylindrical or perforated oral formulations or by using radial gradients of erosion or diffusion-controlling excipients. The ability to produce unique, individual or multi-drug and/or multi-dose formulations, can be the other important advantage of 3DP medicines, as demand for personalised medicine is increasing and becoming a megatrend.

Although research in the field started in the early 1990's, the FDA has approved only a single 3D printed drug since 2015: Spritam^{®}, an orodispersible dosage form obtained by binder jetting method 3DP, and having the following excipients: colloidal silicon dioxide, glycerin, mannitol, microcrystalline cellulose, polysorbate20, povidone, sucralose, butylated hydroxyanisole, natural and artificial spearmint flavor.

**The** 3DP methods that were adapted to handle pharmaceutical agents and biocompatible materials are: Extrusion printing (in particular Fused Deposition Modeling (FDM), pressure assisted microsyringe (PAM), and more recently, direct powder extrusion), Binder jetting (BJ), Material jetting (MJ), Stereolithography (SLA), Selective laser sintering (SLS). For more information about the 3DP classification, mention may be made of publication of reference lon-Bogdan Dumitrescu et al. "The age of pharmaceutical 3D printing technological and therapeutical implications of additive manufacturing. Farmacia, 2018, Vol. 66, 3 (365-389)", in particular Table I.

Extrusion printing, specifically FDM, is the most widely used method in the 3D printing field and is characterized by a pre-heated polymer filament or a semisolid being extruded through a nozzle in precise locations on a platform, using xyz positioning. Once the first layer cools down, the following one is applied. Depending on the shape of the printed product, a different material may be needed to provide structural support until the object cools down completely and solidifies, but afterwards it can be removed. PAM technology is similar, except that the printer is fed with non-melted, viscous materials. It is particularly useful for 3D Bioprinting. 3D Bioprinting refers to methods that employ living cells. While the main advantage of biomedical manufactured scaffolds is the proliferation of living cells, from a pharmaceutical point of view they can also be a source for drug delivery, with antimicrobial drug-eluting implants as an example. Finally, mention can be made of direct powder extrusion, a technology that was recently developed. According to this technology, the printable powder is directly extruded thus avoiding the need for preparation of filaments.

The common 3D printing process chain includes the following step:
- Creation or adjustment of a CAD model, using specialised software, a 3D scan of an already existing object or medical imagery. The CAD file describes the geometry and size of the 3D structure.
- Conversion to one of the printer-ready STL or AMF file formats. These files describe the surface of the model in triangulated sections, depending on the surface curvature degree. Increasing the number of the triangulated sections will increase the resolution of the printed piece.
- Slicing the 3D model into layers of specified thickness using specialist software that can also determine the amount and position of support material, which prevents the collapsing of the printed object. The infill density of the printed object has to be specified as well - with 0% for a hollow structure and with 100% for a solid fill part.

- Transfer to and setting up of the files on the actual 3D printer.
- Layer-by-layer manufacturing of the 3D product.
- Occasionally, drying or removal and discarding of support material and postprocessing are required.

Although 3DP represents a promising technology in the pharmaceutical field, there are still a limited variety of available, environmentally friendly, edible and printer-friendly materials, which is a bottleneck to the wide-scale adoption of 3D printing technologies. The most commonly used pharmaceutical grade polymers as a drug carrier for the manufacture of dosage forms by FDM are polyvinylpyrrolidone (PVP), polyvinyl alcohol (PVA), and polylactic acid (PLA), which are all synthetic fossil oil-based materials.

In line with the current focus on the sustainable economy, the exploring of natural-derived and renewable biopolymers, instead of those fossil oil-based materials, for the fabrication of various products has received tremendous attention.

A variety of natural derived biopolymers such as cellulose, hemicellulose, pectin, starch, alginate, agarose, and chitosan show potential to be utilized in 3D printing of edible dosage forms, owing to their nontoxicity, edibility, high abundancy, bioactivity (e.g. serve as dietary fibers), and long history of utilization in traditional food or medicine production.

However, such biomaterials need to be printable and need to allow the obtaining of a final product having satisfying properties (satisfying mechanical and/or biological and/or pharmacokinetic properties etc.). The problem is even more complex, since according to the 3DP technology employed, the requirements for processability are different.

Among the materials obtained from renewable resources that have already been explored, mention can be made of hydroxypropyl methyl cellulose (HPMC), which has been used notably in FDM 3DP in various studies.

However, the addition of fossil-oil based polymer was always required in order to obtain a printable material, HPMC being in itself, not printable.

Overall, there is an unsatisfied need for printable materials derived from renewable sources, which would allow the obtaining of products, in particular of solid dosage forms, having all required properties.

### Objectives

Therefore, it is an object of the instant invention to provide printable materials and 3DP processes using ingredients obtained from renewable resources.

It is another object of the present invention to provide printable materials which are safe for both the operators and consumers.

It is another object of the present invention to provide printable materials which are easy to handle.

It is another object of the present invention to provide printable compositions and 3DP processes which allow the obtaining of products - in particular of solid dosage forms - having satisfying mechanical properties.

It is another object of the present invention to provide printable materials and 3DP processes allowing the obtaining of products containing active ingredients - in particular solid dosage forms - with satisfying release behavior and/or satisfying pharmacokinetic behavior.

### Presentation of the invention

The inventors found that by combining HPMC with a pregelatinized crosslinked starch compound, printable materials having good printability by HME-3DP could be obtained. In particular, filaments with good mechanical properties could be obtained, which is particularly useful for use in FDM 3DP.

As it will be apparent from the Examples herein after, it can advantageously be used for formulating modified release dosage forms, in particular controlled release dosage forms.

### Summary of the invention

The invention first relates to the use, for Hot Melt Extrusion-based 3D printing (HME-3DP), of a printable material comprising a pregelatinized cross-linked starch and hydroxypropyl methyl cellulose (HPMC).

Preferably, the weight ratio of pregelatinized cross-linked starch compound : HPMC of the printable material is higher than 0.3:1 and lower than 3:1.

Preferably, said printable material further comprises an active ingredient.

Preferably, said printable material further comprises a plasticizer and/or sugar alcohol. Preferably, said sugar alcohol is selected from mannitol, sorbitol, xylitol, or from a mixture thereof.

Preferably, said printable material further comprises an anti-sticking agent.

Preferably, said use is for the HME-3DP of a dosage form. Preferably, said dosage form is a solid dosage form. Preferably, said dosage form is an oral dosage form. Preferably, said dosage form is a controlled release dosage form.

The invention also relates to a process for the manufacture of a product by HME-3DP, comprising the hot melt extrusion a printable material comprising a pregelatinized cross-linked starch and HPMC.

The invention also relates to a filament for HME-3DP, comprising a pregelatinized cross-linked starch and HPMC.

### Brief Description of Drawings

**Fig. 1**

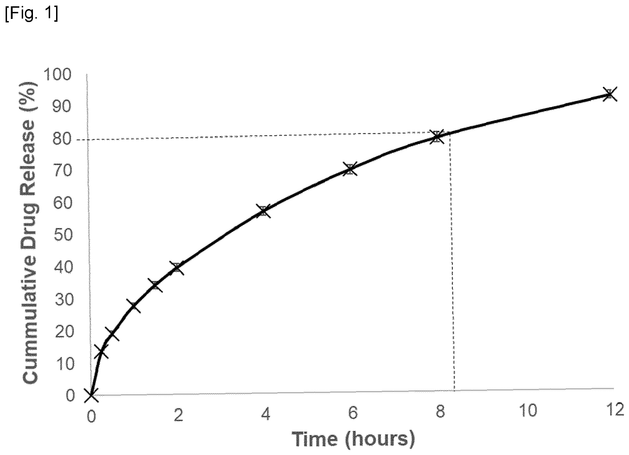

   [Fig. 1] shows the release profile of 3D printed dosage forms according to the disclosure containing the water-soluble active ingredient dyprophilline.

### Description of Embodiments

The invention first relates to the use, for Hot Melt Extrusion based 3D printing (HME-3DP) of a printable material comprising hydroxypropyl methyl cellulose (HPMC) and a starch compound, wherein said starch compound is pregelatinized and crosslinked.

The expression "printable material" refers to a material which can be used as such in a 3DP process, or with the addition of other ingredients. In other words, it can be a ready-to-use material for feeding a 3D printer or a material which can be used in the preparation of a feed for a 3D printer. The printable material according to the disclosure is preferably in the solid state. It is preferably a powder, a granule or a filament. The printable powder can be used as a powder in 3DP (like in direct powder extrusion), or can be used in the preparation of a printable materials in another form (granules, filaments etc.). Preferably, the printable material according to the disclosure is a powder or a filament, still preferably a filament.

The expression "starch compound" classically refers to a substance obtained from starch. It is reminded that the expression "starch" classically refers to the starch isolated from any suitable botanical source such as maize, tapioca barley by any technique well known to those skilled in the art. Isolated starch typically contains no more than 3% of impurities; said percentage being expressed in dry weight of impurities with respect to the total dry weight of isolated starch. These impurities typically comprise proteins, colloidal matters and fibrous residues. Suitable botanical source includes for instance legumes, cereals, and tubers. The starch compounds according to the disclosure can be derived from any suitable botanical source, including for example legumes (e.g. pea), cereals (e.g. maize, rice, wheat, oat), and tubers (e.g. potato, tapioca).

The pregelatinized cross-linked starch compound according to the disclosure preferably is derived from tuber, preferably from potato.

The pregelatinized starch compound according to the disclosure is crosslinked. It is preferably obtainable or obtained by a cross-linking with a compound selected from sodium metaphosphate, sodium trimetaphosphate, phosphorus oxychloride, adipate, epichlorohydrin or from a mixture thereof. It preferably obtainable or obtained by a cross-linking with sodium metaphosphate, sodium trimetaphosphate, phosphorus oxychloride or from a mixture thereof. It still preferably obtainable or obtained by a cross-linking with sodium trimetaphosphate.

The crosslinked-starch starch compound according to the disclosure is also pregelatinized. Pregelatinization classically means that the starch compound particles no longer present birefringence (lack of crystalline phase) under an optical microscope in polarized light. Pregelatinization can be commonly obtained from birefringent starch or birefringent starch compound, *via* a thermal treatment (50-90°C in general, notably depending on the botanical origin of the starch) in presence of water (also often referred as "cooking") with further drying (after the cooking and/or concomitantly). Other chemical(s) might be used as process aid(s). Pregelatinization can be performed for example by drum-drying. In that case, the starch can be cooked before or during the drum-drying step. It can also be cooked and spray-dried. It can also be obtained by extrusion. Preferably, the crosslinked-starch starch compound according to the disclosure is pregelatinized by cooking and drum-drying.

Preferably, the starch compound according to the disclosure has a cold-water solubility equal to or higher than 10%, said percentage being expressed in dry weight of soluble starch with respect to the total weight of starch. Preferably, this cold-water solubility is equal to or higher than 20%, still preferably equal to or higher than 30%, still preferably equal to or higher than 35%, still preferably equal to or higher than 40%. This solubility can be determined for instance by putting 5 grams of the starch compound in 200 mL distilled water. The dry weight dissolved can be determined after centrifugation, and desiccation of the supernatant. This solubility can be for instance determined according to the detailed protocol as disclosed in the Examples. This cold water-solubility is in general lower than 100 %, even equal to or lower than 90%, even equal to or lower than 80%, even equal to or lower than 70%, even equal to or lower than 60%, even equal to or lower than 50%. It is for example equal to about 42%.

The pregelatinized cross-linked starch compound according to the disclosure preferably has a viscosity in water higher than 10 mPa.s at 20°C, at a shear rate of 100 s⁻¹, as determined on an aqueous solution comprising 10% dry weight of said starch compound. Preferably, the viscosity is determined on a rheometer with a 5 cm 1° cone-plate geometry. This viscosity can be for instance determined according to the detailed protocol as disclosed in the Examples. Preferably, said viscosity is equal to or higher than 50 mPa.s, still preferably equal to or higher than 100 mPa.s, still preferably equal to or higher than 1,000 mPa.s, still preferably equal to or higher than 3,000 mPa.s, still preferably equal to or higher than 5,000 mPa.s, still preferably equal to or higher than 6,000 mPa.s, still preferably equal to or higher than 7,000 mPa.s. Preferably, said viscosity is lower than or equal to 15,000, still preferably equal to or lower than 10,000 mPa.s, still preferably equal to or lower than 9,000 mPa.s, still preferably equal to or lower than 8,000 mPa.s. it is for example equal to about 7,000 mPa.s.

In a preferred embodiment, the pregelatinized cross-linked starch compound according to the disclosure is derived from starch having an amylose content by weight between 0 and 80%; said percentage being expressed in dry weight of amylose with respect to the total dry weight of starch. This amylose content can be determined by the person skilled in the art by way of potentiometric analysis of iodine absorbed by amylose to form a complex. It is preferably equal to or higher than 5%, still preferably equal to or higher than 10%, still preferably equal to or higher than 15%. It is preferably equal to or lower than 70%, still preferably equal to or lower than 50%, still preferably equal to or lower than 40%, still preferably equal to or lower than 30%, still preferably equal to or lower than 25%. It is for example equal to about 20%.

The pregelatinized cross-linked starch compound according to the disclosure might undergoes other chemical and/or physical modification than the preferred ones exposed before, as long as it does not interfere with the desired properties, notably in term of safety and properties of the printable material and printed product obtained thereof. However, and because it appears that it is not necessary in the present invention, the pregelatinized cross-linked starch compound according to the disclosure is preferably no further modified.

Preferably, the pregelatinized cross-linked starch according to the disclosure is complying with the FOOD CHEMICALS CODEX as in force on March 1^{st}, 2021. Preferably, the pregelatinized cross-linked starch according to the disclosure is complying with the Monograph JECFA on Food additives as in force on March 1^{st}, 2021. Preferably, the pregelatinized cross-linked starch according to the disclosure is complying with the US code of Federal regulations 21 CFR, § 172.892 as in force on March 1^{st}, 2021. Preferably, the pregelatinized cross-linked starch according to the disclosure is complying with the Commission Regulation (EU) nr.231/2012 of 9 March 2012 (OJ EC L-83/1 dated 22.03.2012) for Food additives as in force on March 1^{st}, 2021.

Preferably, the pregelatinized cross-linked starch according to the disclosure is a product of CAS N° 55963-33-2.

Pregelatinized cross-linked starches particularly useful are commercially available. Mention can be made for instance of PREGEFLO^{®} PI10 commercialized by the Applicant.

In a preferred embodiment, the amount of pregelatinized cross-linked starch compound of the printable material according to the disclosure is equal to or higher than 1%, still preferably higher than 5%, still preferably higher than 10%, still preferably higher than 15%, still preferably higher than 20%; said percentages being expressed by weight with respect to the total weight of said printable material. It is preferably equal to or lower than 90%, still preferably lower than 70%, still preferably lower than 60%, still preferably lower than 50%, still preferably lower than 40%, still preferably lower than 40%, still preferably lower than 35%, still preferably lower than 30%. It is for example equal to about 25%.

The HPMC according to the disclosure might be any suitable grade, for example selected from K4M, K15M, K100M. It is preferably K4M. It preferably excludes the product AFFINISOL^{™} HPMC HME.

In a preferred embodiment, the amount of HPMC of the printable material according to the disclosure is equal to or higher than 1%, still preferably higher than 5%, still preferably higher than 10%, still preferably higher than 15%, still higher than 20%; said percentages being expressed by weight with respect to the total weight of said printable material. It is preferably equal to or lower than 90%, still preferably lower than 70%, still preferably lower than 60%, still preferably lower than 50%, still preferably lower than 40%, still preferably lower than 35%, still preferably lower than 30%. It is for example equal to about 25%.

Preferably, the HPMC according to the disclosure is a product of CAS N° 9004-65-3.

HPMC particularly useful are commercially available. Mention can be made for instance of HPMC K4M commercialized by SHANDONG.

Preferably, the sum of the amounts of pregelatinized cross-linked starch compounds and of HPMC of the printable material according to the disclosure is equal to or higher than 1%, still preferably higher than 5%, still preferably equal to or higher than 10%, still preferably higher than 20%, still higher than 30%, still preferably higher than 40%, still preferably equal to or higher than 45%, still preferably higher than 45%; said percentages being expressed by weight with respect to the total weight of said printable material. It is preferably equal to or lower than 99%, still preferably lower than 90%, still preferably lower than 70%, still preferably lower than 75%, still preferably lower than 60%, still preferably equal to or lower than 55%, still preferably lower than 55%. It is for example equal to about 50%.

Preferably, the weight ratio of pregelatinized cross-linked starch compound : HPMC of the printable material according to the disclosure is higher than 0.3:1 and lower than 3:1. It is preferably equal to or higher than 0.4:1, still preferably equal to or higher than 0.5:1, still preferably equal to or higher than 0.6:1, still preferably equal to or higher than 0.7:1, still preferably equal to or higher than 0.8:1 still preferably higher than 0.9:1. It is preferably equal to or lower than 2.5:1, still preferably equal to or lower than 2:1, still preferably equal to or lower than 1.8:1, still preferably equal to or lower than 1.6:1, still preferably equal to or lower than 1.4:1, still preferably equal to or lower than 1.2:1. It is for example equal to about 1:1.

In general, the printable material according to the disclosure further includes other ingredients than the pregelatinized cross-linked starch compound and the HPMC according to the disclosure. Examples of such other ingredients are: active ingredients; sugars; sugar alcohol; colors; flavors; lubricants; other starches and starch compounds. Other examples are excipients classically used in 3D printing, in particular in FDM, like polylactic acid (PLA), cellulose acetate hydroxypropylcellulose (HPC), ethyl cellulose (EC), hydroxypropyl methylcellulose acetate succinate (HPMCAS), polyvinylpyrrolidone sigma (PVP), crospovidone, microcrystalline cellulose (MCC), Carbopol^{®} 794, polyethylene oxide , polyethylene glycol, Eudragit^{®}, polycaprolactone (PCL), triethyl citrate, tri-calcium phosphate, poly(vinyl alcohol) (PVA), Soluplus^{®}, Kollidon^{®}, Kollicoat^{®}, Polyox^{™}. Although the use of fossil-oil based excipients like PLA, PVP, crospovidone, Carbopol^{®} 794, polyethylene oxide, polyethylene glycol, Eudragit^{®}, PCL, triethyl citrate, tri-calcium phosphate, PVA, Soluplus^{®}, Kollidon^{®}, Kollicoat^{®}, or Polyox^{™} is preferably excluded.

Preferably, the printable material according to the disclosure further comprises an active ingredient. The active ingredient according to the disclosure includes non-pharmaceutical and pharmaceutical agents. The expression "active" classically refers to any substance of pharmaceutical, veterinary, food, nutraceutical, cosmetic or agrochemical interest. Preferably, the active ingredient according to the disclosure is a pharmaceutical, nutraceutical, cosmetic or veterinary active ingredient. The active ingredient, in particular when it is a pharmaceutical active ingredient, may be chosen from so-called small molecules, but also from so-called "biological" active ingredients, as is the case for example of active substance based on or derived from proteins, nucleic acids - such as those derived from DNA or RNA - cells or viruses. Preferably, the active ingredient of the disclosure is diprophylline.

The active ingredient according to the disclosure might be very soluble in water, freely soluble in water, soluble in water, sparingly soluble in water, slightly soluble in water, very slightly soluble in water or practically insoluble in water. This solubility in water is well defined for instance in 9th edition of The International Pharmacopeia (2019), Section "General Notice, Solubility". In the instant disclosure, the expression "water-insoluble" classically refers to active ingredients which are from sparingly soluble in water to practically insoluble in water. In the instant disclosure, the expression "water-soluble" classically refers to active ingredients which are from soluble in water to very soluble in water.

Preferably, the active ingredient according to the disclosure is water-soluble, still preferably freely soluble in water or very soluble in water. It is preferably diprophylline, which is freely soluble in water.

In general, the amount of active ingredient of the printable composition is equal to or higher than 1%, even equal to or higher than 2%, even equal to or higher than 5%, even equal to or higher than 10%, even equal to or higher than 15%; said percentages being expressed by weight with respect to the total weight of said printable material. It is in general equal to or lower than 99%, even equal to or lower than 90%, even equal to or lower than 70%, even equal to or lower than 50%, even equal to or lower than 40%, even equal to or lower than 30%, even equal to or lower than 20%, even equal to or lower than 25%. It is for example equal to about 20%.

Preferably the printable material according to the disclosure further comprises a sugar alcohol or a plasticizer. Plasticizers and sugar alcohols according to the disclosure preferably are selected from glycerol, sorbitol, sorbitol anhydrides, maltitol, mannitol, xylitol, polyethylene glycol with a molecular weight of between 400 and 10 000 daltons, polyethylene glycol stearate, propylene glycol, triethyl citrate, acetyl triethyl citrate, tributyl citrate, polysorbate, acetylated monoglycerides, lactic acid esters, fatty acids and their salts or derivatives which are ethoxylated, such as, in particular, stearic acid, phthalates, ethyl sebacate, butyl sebacate, miglyol, glycerol triacetate, liquid paraffin, lecithin, carnauba wax, hydrogenated castor, urea, or from a mixture thereof. It is preferably selected from sugar alcohols, preferably from mannitol, sorbitol, or from a mixture thereof.

Preferably, the amount of plasticizer or sugar alcohol in said printable material is equal to or higher than 1%, preferably higher than 5%, still preferably higher than 8%, still preferably higher than 10%, still preferably higher than 15%, still preferably higher than 18%, still preferably equal to or higher than 20%, still preferably equal to or higher than 23%, still preferably higher than 23%; said percentages being expressed by weight with respect to the total weight of said printable material. It is preferably equal to or lower than 80%, still preferably lower than 70%, still preferably lower than 60%, still preferably lower than 50%, still preferably lower than 48%, still preferably lower than 38%, still preferably lower than 35%, still preferably equal to or lower than 33%, still preferably lower than 33%.

When a mixture of mannitol and sorbitol is used, the sorbitol: mannitol weight ratio is preferably higher than 1:1, still preferably equal to or higher than 2:1. It is preferably equal to or lower than 6:1, still preferably equal to or lower than 5:1, still preferably equal to or lower than 4:1. It is for example of about 3:1.

Preferably, this printable material further comprises an anti-sticking agent, preferably selected from meltable thermal lubricants for instance from metallic salts of fatty acids, fatty acids, fatty alcohol, fatty acid esters hydrocarbons, or form a mixture thereof. It is for example selected from glyceryl monostearate, magnesium stearate, calcium stearate, sodium stearate, hexadecane, octadecane, sodium stearyl fumarate, glyceryl behenate, acid stearic, or form a mixture thereof. It preferably comprises stearic acid. It is still preferably stearic acid alone. Preferably, the amount of anti-sticking agent is between 0.5 and 5%, preferably between 1 and 4%, preferably between 1 and 3%, for example equal to about 2%; said percentages being expressed by weight with respect to the total weight of said printable material.

Preferably, the printable material according to the disclosure is composed of:
- from 1 to 96.5% of pregelatinized cross-linked starch compound;
- from 1 to 96.5% of HPMC;
- from 1 to 96.5% of active ingredient;
- from 1 to 80% of plasticizer or sugar alcohol, preferably of a mixture of mannitol and sorbitol;
- from 0.5 to 5% of anti-sticking agent;
- from 0 to 95.5% of other ingredients;
said percentages being expressed by weight with respect to the total weight of said printable material and their sum being equal to 100%.

Preferably, the amount of other ingredients of this printable material is lower than 90%, still preferably lower than 50%, still preferably lower than 30%, still preferably lower than 10%, still preferably lower than 5%, still preferably lower than 1%; said percentages being expressed by weight with respect to the total weight of said printable material. Still preferably, this printable material is devoid of such other ingredients.

Preferably, the amount of ingredients obtained from renewable sources in the printable material is equal to or higher than 50%, said percentage being expressed by weight with respect to the total weight of said printable material. Such ingredients obtained from renewable sources are in particular not obtained from fossil-oils. Still preferably, this amount is equal to or higher than 60%, still preferably equal to or higher than 70%, still preferably equal to or higher than 80%, still preferably equal to or higher than 90%, still preferably equal to 100%. Examples of ingredients which can be obtained from renewable sources are starch compounds - including the pregelatinized cross-linked starch compound according to the disclosure - cellulose derivatives - including the HPMC according to the disclosure - sugars and sugar alcohols. Example of ingredients which cannot be obtained from renewable sources are PLA, PVP, crospovidone, Carbopol^{®} 794, polyethylene oxide, polyethylene glycol, Eudragit^{®}, PCL, triethyl citrate, tri-calcium phosphate, PVA, Soluplus^{®}, Kollidon^{®}, Kollicoat^{®}, or Polyox^{™}.

Preferably, when an active ingredient is present, the excipients of the printable material according to the disclosure are composed of at least 50% of ingredients obtained from renewable sources, in particular not obtained from fossil-oils; said percentage being expressed by weight with respect to the total weight of excipients of said printable material. Still preferably, the excipients of the printable material according to the disclosure are composed of at least 60% by weight of ingredients obtained from renewable sources, still preferably of at least 70%, still preferably of at least 80%, still preferably of at least 90%, still preferably of 100%.

The printed product or product to be printed according to the disclosure typically is a solid or semi-solid product, preferably intended for oral administration, for instance a tablet, a gel, a film, a caplet, a softgel, a hard capsule. It can also be a drug loaded medical device. In a preferred embodiment, the printed product or product to be printed according to the disclosure is a solid product, typically a tablet. In a preferred embodiment, the printed product according to the disclosure is not a gel and/or not a film. The printed product or product to be printed according to the disclosure can be for instance of pharmaceutical, veterinary, nutraceutical, food, cosmetic, or agrochemical interest. It can be for humans or animals. It is preferably a pharmaceutical, veterinary, nutraceutical or cosmetic product. In a preferred embodiment, the printed product or product to be printed according to the disclosure is a dosage form (i.e. a product comprising an active ingredient), preferably a solid dosage form, preferably an oral solid dosage form, in particular a pharmaceutical oral solid dosage form.

Preferably, the printed product according to the disclosure is a modified release dosage form, preferably a controlled release dosage form. Preferably, the modified or controlled release property of the dosage form, is determined according to the guidance as provided by the US FDA (General Methods; 32(2) Second Interim Revision Announcement: DISSOLUTION, <711> DISSOLUTION; 11/21/2016), using dissolution method A (pH transition method, simulated gastric fluid for 2 hours then change to simulated intestinal fluid for the remaining 10 hours). For a modified release dosage form, 80% by dry weight or less of the active ingredient should be released in 30 minutes, as opposed to instant release dosage forms. For controlled release dosage forms, 80% by dry weight or less of the active ingredient should dissolve in 8 hours. Of course, the active ingredient must still be released. Therefore, preferably, at least 20% by dry weight of the active ingredient should dissolve in 8 hours. Still preferably, the controlled release dosage form according to the disclosure releases at least 30% by dry weight of the active ingredient, still preferably at least 40%, still preferably at least 50%, still preferably at least 60%, still preferably at least 80%.

**The** HME-3DP can be performed by any suitable method known to those skilled in the art. Preferably, 3D printing is carried out by a method selected from fused deposition modeling (FDM) or direct powder extrusion, preferably FDM.

The invention also covers a product obtained or obtainable by HME-3DP, comprising a pregelatinized cross-linked starch compound and HPMC, said starch compound and HPMC being preferably as described before in the specification. Preferably, said printed product is a described before in the specification. Preferably, the printed product of the instant disclosure has the composition as described before for the printable material, with the exception of the eventual added solvents or moisture of the ingredients used, which are ignored.

The invention also covers a process for the manufacture of a product by HME-3DP, comprising the hot melt extrusion of the printable material such as defined before. Preferably, the printed product or product to be printed is as defined before. Preferably, the HME-3DP is performed by a method selected from fused deposition modeling (FDM) or direct powder extrusion, preferably FDM.

In the instant disclosure, the expression "between" classically includes the recited upper and lower limits.

In the instant disclosure, the amounts of ingredients are generally expressed in percentages by weight. Unless otherwise specified these weights are amounts of ingredients in their original form, which is generally in powder form. These powdery ingredients generally include small amount of water (also referred as %moisture or as "loss on drying"). With that respect: starch compounds according to the disclosure generally contain no more than 15% by weight of water, generally 5 to 10%; sugar alcohols like mannitol and sorbitol generally contain no more than 1% water. This means that when it is for example referred to 10% by weight of a starch compound, this generally corresponds to 9.0 to 9.5% in dry weight (i.e. anhydrous weight).

By opposition, in the instant disclosure, when amounts are expressed in dry weight, the anhydrous weight is taken into consideration.

Other characteristics and advantages of the present invention will emerge clearly on reading the examples given hereinafter, which illustrate the invention without however limiting it.

### Examples

### A. Materials

### A.1. Starch compound used and characterization thereof.

Viscosity measurement. Measurements were performed on a rheometer (Physica MCR301, Anton Paar) with a 5 cm 1° cone-plate geometry (CP50). The temperature was regulated by way of Peltier. The measurements were performed at 20°C on an aqueous solution comprising 10% dry starch compound as follow: 1 minute equilibrium at 20°C; shear rates from 0.006 to 1,000 s⁻¹(log) applied in 3 minutes at 20°C.

Solubility measurement. In a 250 ml beaker, 200 ml of distilled water were added. 5 grams weigh of starch compound were added and the mixture was homogenized by magnetic agitation for 15 minutes. The resulting solution / suspension was centrifuged 10 minutes at 4,000 rpm. 25 mL of the supernatant were taken out and introduced into a crystallizer and place into an oven at 60°C, until the water evaporates. It was then placed into an oven at 103+C ± 2°C for 1 hour. The residue was placed into a desiccator to cold down at room temperature and then weighted for calculating the solubility in dry weight.

The following starch compound was used: pregelatinized cross-linked starch cross-linked with sodium trimetaphosphate (PREGEFLO^{®} PI10, ROQUETTE), having a moisture of 6.0% by weight, a solubility in water of 42%, and a viscosity (10%, 20°C, 100 s⁻¹) of 7,090 mPa.s.

### A.2. Other ingredients used

The following other ingredients were used: HPMC K4M (SHANDONG), having a moisture of about 5% by weight; sorbitol (NEOSORB^{®} 100C, ROQUETTE), mannitol (PEARLITOL^{®} 100 SD, ROQUETTE), diprophylline (Shanghai Star), stearic acid (Stearic acid 50, SIGMA-ALDRICH).

### B. Preparation of controlled release tablets comprising a water-soluble active ingredient by FDM

In this Example, the inventors tested the impact of the use of a pregelatinized cross-linked starch in combination with HPMC for HME-3DP.

The inventors prepared filaments from various formulations, as shown in Table 1.

**[Table 1]**

| Ref | F1 | F2 | F3 |
|---|---|---|---|
| pregelatinized cross-linked starch (PREGEFLO^{®} PI10) | 25% | 50% | / |
| HPMC K4M | 25% | / | 50% |
| Active ingredient (diprophylline) | 20% | 20% | 20% |
| Sorbitol | 28% | 21% | 21% |
| Mannitol | 0% | 7% | 7% |
| Stearic acid | 2% | 2% | 2% |

In this table, percentages are expressed by weight, with respect to the total weight of the printable material (powder mix used to prepare the filaments).

Ingredients were sieved through 500µm sieve and blending was done at 40rpm. For preparing filaments, the blend was then passed through a twin-screw extruder, which consist of 4 zones (from feeding zone, mixing, kneading and exit): the temperature for each zone was: 120°C, 160°C, 160°C and 120°C.

Printing was done using Ultimaker S5 printer with printhead BB core (mainly for water soluble materials) at 180°C, speed is at 4mm/s, and 0.065mm as print height. Tablets to be printed were cylindrical tablets of 10mm diameter and 5mm height.

Only the filaments obtained from formulation F1 could achieve a continuous printing. Formulation F3, devoid of the starch compound according to the disclosure, was not extrudable, such that no filaments was formed. Formulation F2, devoid of HPMC, allowed the obtaining of filaments, but they were too brittle to be printed.

The tablets (at least 6 tablets) obtained from formulation F1 were tested for their controlled release properties according to the guidance as provided by the US FDA (General Methods; 32(2) Second Interim Revision Announcement: DISSOLUTION, <711> DISSOLUTION; 11/21/2016),using dissolution method A (pH transition method, simulated gastric fluid for 2 hours then change to simulated intestinal fluid for the remaining 10 hours).

The release profile obtained is shown in Figure 1. 79% by dry weight of the active ingredient was released in 8 hours.

These results first show that the combination according to the disclosure (i.e. the combination of pregelatinized cross-linked starch compound and of HMPC) is printable. It allows the obtaining of filament with good mechanical properties. It also shows that the combination according to the disclosure can advantageously be used for formulating controlled release dosage forms.

**The** tablets (at least 6 tablets) obtained from formulation 1 were also tested for their stability. A 3-months stability study shown that no significant changes were observed regarding the release profile of the active ingredient, in 2 storage conditions (i.e. 25°C / 60% Relative Humidity and 40°C / 75% Relative Humidity).

## Claims

1. Use, for Hot Melt Extrusion-based 3D printing (HME-3DP), of a printable material comprising a pregelatinized cross-linked starch and hydroxypropyl methyl cellulose (HPMC).

2. The use of claim 1, wherein the weight ratio of pregelatinized cross-linked starch compound : HPMC of the printable material is higher than 0.3:1 and lower than 3:1.

3. The use of any of claims 1 or 2, wherein said printable material further comprises an active ingredient.

4. The use of any of claims 1 to 3, wherein said printable material further comprises a plasticizer and/or sugar alcohol.

5. The use of claim 4, wherein said sugar alcohol is selected from mannitol, sorbitol, xylitol, or from a mixture thereof.

6. The use of any of claims 1 to 5, wherein said printable material further comprises an anti-sticking agent.

7. The use of any of claims 1 to 6, for the HME-3DP of a dosage form.

8. The use of claim 7, wherein said dosage form is a solid dosage form.

9. The use of any of claims 7 or 8 wherein said dosage form is an oral dosage form.

10. The use of any of claims 7 to 9 wherein said dosage form is a controlled release dosage form.

11. A process for the manufacture of a product by HME-3DP, comprising the hot melt extrusion a printable material comprising a pregelatinized cross-linked starch and HPMC.

12. A filament for HME-3DP, comprising a pregelatinized cross-linked starch and HPMC.
